# EUROPEAN PATENT SPECIFICATION

(11) **EP 2 563 230 B1**
(45) Date of publication and mention of the grant of the patent: **12.10.2016**
(21) Application number: 11775368.1
(22) Date of filing: 18.04.2011
(51) Int. Cl.: A61B 17/00, A61B 10/02

(54) **BIOPSY EXTRACTION UNIT**
BIOPSIEEXTRAKTIONSEINHEIT
UNITÉ D'EXTRACTION DE BIOPSIE

(30) Priority: 27.04.2010 SE 1050419
(43) Date of publication of application: 06.03.2013
(73) Proprietor: AprioMed AB, 754 50 Uppsala (SE)
(72) Inventor: ÅKERFELDT, Dan, 741 91 Knivsta (SE); ÅSTRÖM, Gunnar, 752 31 Uppsala (SE)
(74) Representative: Brann AB
(86) International application number: PCT/SE2011/050475
(87) International publication number: WO 2011/136719

(56) References cited:
- WO-A2-2007/021905
- DE-A1- 10 026 303
- US-A- 3 007 471
- US-A- 4 917 100
- US-A- 5 509 923
- US-A1- 2008 139 961
- US-B1- 6 419 641
- US-B1- 6 443 910
- US-B1- 6 443 910

## Description

### Field of the invention

The present invention relates to biopsy extraction unit, according to the preambles of the independent claims.

### Background of the invention

In the prior art, as exemplified by US-6,063,037; US-6,755,793; US-7,033,324, and US-6,443,910, an outer cannula used for biopsy is provided with an inner cannula slidable within the outer cannula and used to grasp the tissue when withdrawing the outer cannula. Often the inner cannula is provided with longitudinal slits in the distal end that cooperates with the inner conical shape of the outer cannula in order to grasp and hold the tissue sample when the entire biopsy needle is withdrawn.

DE 10026303A1 discloses another type of biopsy device comprising a rod with different cross-sectional shapes arranged within an outer cannula, creating channels for suction to be applied to a tissue sample site. Yet another type of biopsy device is shown in US-3,007,471, comprising an inner cutting stylet arranged within an outer cannula. The inner cutting stylet blade is used to sever a tissue sample from a tissue site.

It is sometimes desired to have a biopsy needle with a small outer diameter and when reducing the outer diameter of the biopsy needle the inner needle provided with longitudinal slits often is difficult to manufacture and use due to the small dimensions. E.g. the risk increases that the inner needle will be deformed during use.

The inventor has identified that problem, in particular when using small inner cannulas provided with slits.

Traditional biopsy devices used today have an outer cannula having an outer diameter of 3-4 mm and has a material thickness of approximately 10% of that diameter, i.e. 0.3 - 0.4 mm. The distal end of such a biopsy device has often an inner conical shape in order to reduce the friction to the tissue sample which facilitates the sample to be pressed into the tube of the biopsy device. Due to the conical shape the inner diameter at the distal end is approximately 15 % less then the inner diameter of the rest of the cannula.

As an example, a biopsy device having an outer cannula with an outer diameter of 3 mm will then have an inner diameter of 2.4 mm and an inner diameter at the very distal end being approximately 2.0 mm. This will also be the diameter of the tissue sample. In this case an inner cannula provided with slits would have a material thickness of approximately 0.15 mm to be inserted into the outer cannula without destroying the tissue sample. Furthermore, there is a risk that the distal end of the inner cannula that is provided with the slits would be deformed and the inner cannula must then be discarded after only been used once.

There is an ongoing trend towards biopsy devices having smaller diameters mainly in order to reduce the trauma during the sampling procedure. In order to still be able to take tissue samples having the same size, e.g. diameter, the material thicknesses of both the outer and inner cannulas must be reduced. In addition, the inner conical shape of the outer cannula will be less, which in turn have negative impacts of the inner cannula's ability to firmly hold the tissue sample. This results in that the space for an inner cannula with slits is reduced and that its material thickness will be so thin that it is very difficult to manufacture and to handle it without being deformed.

Thus, an inner cannula provided with slits requires a reduction of the inner diameter at the outer cannula's distal end in order for the inner cannula to work as intended. The inner cannula requires some free space around the tissue sample when it is inserted in order not to expel the sample when the inner cannula is inserted. The diameter reduction results in that the distal end of the inner cannula is forced inwards in the radial direction and locks the tissue sample inside the biopsy device tube. The distal tip of the inner cannula may then be deformed and unusable after only one tissue sampling.

The object of the present invention is to solve the problems described in relation to tissue sampling, and in particular in relation to tissue sampling using biopsy devices having smaller outer diameters.

### Summary of the invention

The above-mentioned object is achieved by the present invention according to the independent claim.

Preferred embodiments are set forth in the dependent claims.

Thus, according to present invention the above problem is solved by using a biopsy extraction unit provided with a sample rod that is inserted into the outer cannula such that the sample rod is inserted along the inner wall of the outer cannula, i.e. between the inner wall and the tissue sample, and thereby holding the tissue within the biopsy tube.

The sample rod has a diameter that may be several times larger than the material thickness of a presently used inner cannula and is thereby easier to manufacture and there is no risk that the sample rod will be deformed during use. The biopsy extraction unit may therefore be used several times. In addition, the reduction of the inner diameter of the outer cannula is not required for achieving the tissue sampling.

The sample rod has a very little surface being in contact with the tissue sample when the rod is inserted which results in that the friction between the sample rod and the tissue sample is considerably low. The tissue sample is held, or locked, within the outer cannula due to the radial forces that the sample rod exerts on the sample tissue that is pressed towards the relatively large inner surface opposite the position of the sample pin. The tissue sample is held in place by the friction of the inner surface of the outer cannula during withdrawal of the biopsy device.

### Short description of the appended drawings

Figure 1 is a side view of the biopsy extraction unit according to a first embodiment of the present invention.
Figure 2 is a side view of the biopsy extraction unit according to a second embodiment of the present invention.
Figures 3-5 are cross-sectional side views illustrating the use of the biopsy extraction unit in relation to a biopsy device.
Figure 6 is a cross-sectional view of A-A in figure 5 of the biopsy extraction unit in relation to the biopsy device according to one embodiment.
Figure 7 is a cross-sectional view of A-A in figure 5 of the biopsy extraction unit in relation to the biopsy device according to another embodiment.

### Detailed description of preferred embodiments of the invention

The present invention will now be described with references to the appended drawings.

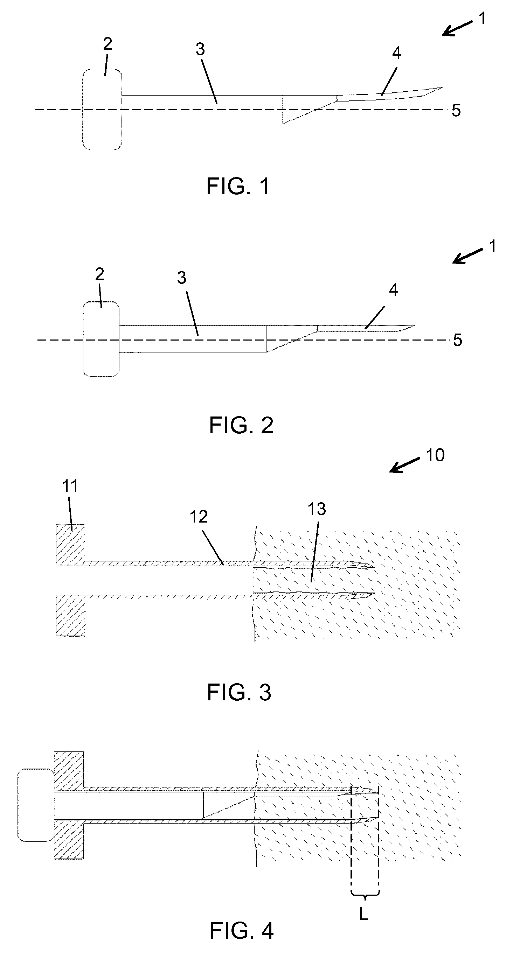

Figure 1 is a side view of the biopsy extraction unit according to a first embodiment of the present invention.

Figure 2 is a side view of the biopsy extraction unit according to a second embodiment of the present invention.

The biopsy extraction unit 1 as illustrated in figures 1 and 2 is provided with a unit handle 2, an intermediate part 3 and an elongated sample rod 4. The extraction unit 1 is adapted for cooperation with a biopsy device 10 provided with an outer cannula 12 which is illustrated in figure 3. The outer cannula 12 is attached to a device handle 2. The distal end of the outer cannula 12 is sharpened in order to facilitate insertion into the tissue in order to take a tissue sample 13 from a desired location. The intermediate part 3 has preferably a circular cross-section and a cross-sectional shape that fits within the outer cannula 12 such that the extraction unit 1 easily may be inserted into the cannula 12. The intermediate part 3 has a guiding purpose, i.e. to position the sample rod 4 in relation to the inner surface of the outer cannula 12 such that the sample rod 4 is positioned along and close to the wall.

The sample rod 4 is offset and essentially parallel to the longitudinal central axis 5 of the biopsy extraction unit 1. The central axis 5 is illustrated by a dashed line in figures 1 and 2.

When using the biopsy extraction unit 1 the sample rod 4 is adapted to essentially be positioned along an inner wall of the outer cannula 12 when fully inserted into the cannula.

The distal tip of the sample rod 4 is sharpened such that the sharpened edge is located furthest away from the central axis 5 in the radial direction. This is clearly illustrated in figures 1 and 2. The reason to sharpen the sample rod 4 in this way is to minimize the influence by the sample rod on the tissue sample 13 when the sample rod 4 is inserted into the outer cannula 12.

In a first embodiment the distal part of the sample rod 4 is bent outwards with regard to the central axis 5. This is illustrated by figure 1. The sample rod has a certain flexibility permitting it to flex in the radial inward direction when inserted into the outer cannula but still exerting a pressure outwards towards the inner surface. In an initial position, i.e. prior the sample rod 4 is inserted into the outer cannula the most distal part of the sample rod 4 is approximately 50-100% farther away from the central axis 5 in the radial direction compared to the most proximal part of the sample rod 4, i.e. the part at the interface towards the intermediate part 3 of the extraction unit 1.

In a second embodiment the sample rod 4 is parallel to the central axis 5 along its entire length. This is illustrated by figure 2.

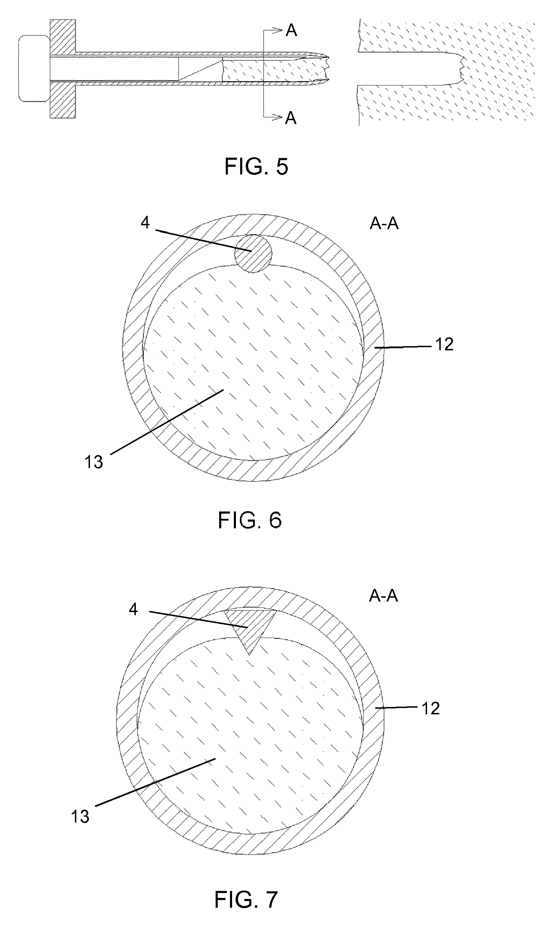

Figure 6 is a cross-sectional view of A-A in figure 5 of the biopsy extraction unit in relation to the biopsy device according to one embodiment.
In this embodiment the sample rod 4 has an essentially circular cross-section.

Figure 7 is a cross-sectional view of A-A in figure 5 of the biopsy extraction unit in relation to the biopsy device according to another embodiment. In this embodiment the sample rod 4 has an essentially triangular cross-section having one side facing outwards in relation to the central axis of the unit.

Further cross-sectional shapes of the sample rod 4 are naturally possible. One important feature is that the rod has a limited surface area in relation to the inner surface area of the outer cannula 12 independent of the cross-sectional shape of the rod. Another important feature is that the rod has a certain width in order to exert radial pressure upon the tissue sample 13 to hold it within the biopsy tube. This width may be in the interval of 3-15% of the surface area, where 15% is a sample rod that covers 15% of the inner surface; in that case the sample rod has a limited thickness. The sample rod 4 illustrated in figure 6 covers approximately 5% of the inner surface.

The maximal cross-sectional extension of the sample rod 4 is naturally related to the outer diameter of the outer cannula 12 and to the material thickness of the cannula wall. Therefore, within the scope defined by the claims, the sample rod 4 may have e.g. elliptical, rectangular, or quadratic cross-sections.

As an example, the maximal extension is in the range of 100-300 % of the thickness of the cannula wall and 10-20 % of the outer diameter of the cannula.

Preferably, at least the sample rod 4 is made from metal, e.g. stainless steel or any suitable alloy.

Alternatively, at least the sample rod 4 is made from plastic. The entire biopsy extraction may be made from plastic and would then be a disposable article.

Figures 3-5 are cross-sectional side views illustrating the use of the biopsy extraction unit 1 in relation to a biopsy device 10. A device handle 2 is attached to the outer cannula 12.

Initially, the outer cannula 12 is inserted into the tissue where a tissue sample 13 is to be taken, this is illustrated in figure 3. During the insertion an obturator (not shown) may be arranged within the cannula in order to prevent tissue from entering the biopsy tube prior to the tissue sample location being reached.

In figure 4 is illustrated the state when the biopsy extraction unit is fully inserted into the outer cannula. It is important that the biopsy extraction unit is used with a mating outer cannula such that the length of the sample rod is such that when the biopsy extraction unit is fully inserted the distal tip of the sample rod is located within the outer cannula. In the figure, L designates the distance between the respective distal tips. L is naturally related to the dimension of the biopsy cannula and may preferably be in the interval of 0 - 3 mm.

In figure 5 the biopsy device including the outer cannula, the biopsy extraction unit and the sample is withdrawn.
Afterwards the tissue sample may be expelled from the biopsy tube in a well-known manner. E.g. by using a suitable rod.

Thus, the method of biopsy tissue sampling, comprises the following steps:
- inserting an outer cannula into tissue where a tissue sample is to be taken,
- inserting a sample rod of a biopsy extraction unit, as described above, into the outer cannula, and
- withdrawing the outer cannula with the sample rod.

The present invention is not limited to the above-described preferred embodiments. Various alternatives, modifications and equivalents may be used. Therefore, the above embodiments should not be taken as limiting the scope of the invention, which is defined by the appending claims.

## Claims

1. Biopsy extraction unit (1) for use in relation to a biopsy device (10) provided with an outer cannula (12), wherein
the biopsy extraction unit (1) is provided with a unit handle (2), and an intermediate part (3), wherein the biopsy extraction unit (1) further comprises an elongated sample rod (4), wherein the sample rod (4) is adapted to essentially be positioned along an inner wall of the outer cannula (12) when inserted into said biopsy device (10),
wherein the rod (4) is arranged distally of the intermediate part (3),
the rod (4) being offset and essentially parallel to a longitudinal central axis (5) of the biopsy extraction unit (1),
**characterized in that** the sample rod (4) has a circular, elliptical, or quadratic cross-section, or wherein the sample rod (4) has a triangular cross-section having one side facing outwards in relation to the central axis (5) of the unit (1) or wherein the sample rod (4) has a rectangular cross-section having one side facing outwards in relation to the central axis (5) of the unit (1), and
wherein the sample rod (4) is configured to exert radial pressure on a tissue sample (13) contained within the outer cannula (12) to hold said sample (13) within said cannula (12) when said sample rod (4) is inserted into said biopsy device (10).

2. Biopsy extraction unit (1) according to claim 1, wherein the distal tip of the sample rod (4) is sharpened such that the sharpened edge is located furthest away from the central axis (5) in the radial direction.

3. Biopsy extraction unit (1) according to any preceding claim, wherein the sample rod (4) is parallel to the central axis (5) along its entire length.

4. Biopsy extraction unit (1) according to any preceding claim, wherein the distal part of the sample rod (4) is bent outwards.

5. Biopsy extraction unit (1) according to any preceding claim, wherein at least the sample rod (4) is made from metal.

6. Biopsy extraction unit (1) according to any of claims 1-4, wherein at least the sample rod (4) is made from plastic.

7. Biopsy device comprising a biopsy extraction unit (1) according to any preceding claim, and further comprising an outer cannula (12), wherein the length of the sample rod (4) is such that when the biopsy extraction unit (1) is fully inserted into the outer cannula (12) the distal tip of the sample rod (4) is located within the outer cannula (12).

8. Biopsy device according to claim 7, wherein the sample rod (4), when inserted into the outer cannula (12), covers 3-15 % of the inner surface area of the outer cannula (12).

9. Biopsy device according to claim 7 or 8, wherein the sample rod (4) has a maximal cross-sectional width in the interval of 100-300 % of the thickness of the cannula wall of said outer cannula (12), and wherein the sample rod (4) has a maximal cross-sectional width in the interval of 10-20 % of the outer diameter of said outer cannula (12).

10. Biopsy device according to any of claims 7-9, wherein a distance (L) between the distal tip of the sample rod (4) and the distal tip of the outer cannula (12) is in the interval of 0 - 3 mm, when the biopsy extraction unit (1) is fully inserted into the outer cannula (12).

## Patentansprüche

1. Biopsie-Extraktions-Einheit (1) zur Verwendung in Bezug auf eine Biopsie-Vorrichtung (10), ausgestattet mit einer äußeren Kanüle (12), wobei die Biopsie-Extraktions-Einheit (1) mit einem Einheits-Griff (2) und einem Zwischenteil (3) ausgestattet ist, wobei die Biopsie-Extraktions-Einheit (1) weiterhin eine gestreckten Probenstange (4) umfasst, wobei die Probenstange (4) angepasst ist, um im Wesentlichen entlang einer Innenwand der äußeren Kanüle (12) positioniert zu sein, wenn in die Biopsie-Vorrichtung (10) eingeschoben, wobei die Stange (4) distal von dem Zwischenteil (3) angeordnet ist,
die Stange (4) versetzt und im Wesentlichen parallel zu einer längslaufenden Mittelachse (5) der Biopsie-Extraktions-Einheit (1) ist,
**dadurch gekennzeichnet, dass** die Probenstange (4) einen kreisförmigen, elliptischen oder quadratischen Querschnitt aufweist, oder wobei die Probenstange (4) einen dreieckigen Querschnitt mit einer Seite aufweist, die in Bezug auf die Mittelachse (5) der Einheit (1) auswärts weist oder wobei die Probenstange (4) einen rechteckigen Querschnitt mit einer Seite aufweist, die in Bezug auf die Mittelachse (5) der Einheit (1) auswärts weist, und
wobei die Probenstange (4) so ausgelegt ist, dass Radialdruck auf eine in der äußeren Kanüle (12) enthaltenen Gewebeprobe (13) ausgeübt wird, um die Probe (13) in der Kanüle (12) zu halten, wenn die Probenstange (4) in die Biopsie-Vorrichtung (10) eingeschoben wird.

2. Biopsie-Extraktions-Einheit (1) nach Anspruch 1, wobei die distale Spitze der Probenstange (4) derart geschärft ist, dass die geschärfte Kante am weitesten weg von der Mittelachse (5) in der radialen Richtung angeordnet ist.

3. Biopsie-Extraktions-Einheit (1) nach einem vorangehenden Anspruch, wobei die Probenstange (4) entlang ihrer gesamten Länge parallel zu der Mittelachse (5) ist.

4. Biopsie-Extraktions-Einheit (1) nach einem vorangehenden Anspruch, wobei der distale Teil der Probenstange (4) auswärts gebogen ist.

5. Biopsie-Extraktions-Einheit (1) nach einem vorangehenden Anspruch, wobei mindestens die Probenstange (4) aus Metall hergestellt ist.

6. Biopsie-Extraktions-Einheit (1) nach einem der Ansprüche 1-4, wobei mindestens die Probenstange (4) aus Kunststoff hergestellt ist.

7. Biopsie-Vorrichtung, umfassend eine Biopsie-Extraktions-Einheit (1) nach einem vorangehenden Anspruch und weiterhin umfassend eine äußere Kanüle (12), wobei die Länge der Probenstange (4) derart ist, dass wenn die Biopsie-Extraktions-Einheit (1) vollständig in die äußere Kanüle (12) eingeschoben ist, die distale Spitze der Probenstange (4) in der äußeren Kanüle (12) angeordnet ist.

8. Biopsie-Vorrichtung nach Anspruch 7, wobei die Probenstange (4), wenn in die äußere Kanüle (12) eingeschoben, 3-15 % der inneren Oberfläche der äußeren Kanüle (12) erfasst.

9. Biopsie-Vorrichtung nach Anspruch 7 oder 8, wobei die Probenstange (4) eine maximale Querschnittsbreite in dem Intervall von 100-300 % der Dicke der Kanülenwand der äußeren Kanüle (12) aufweist, und wobei die Probenstange (4) eine maximale Querschnittsbreite in dem Intervall von 10-20 % des äußeren Durchmessers der äußeren Kanüle (12) aufweist.

10. Biopsie-Vorrichtung nach einem der Ansprüche 7-9, wobei ein Abstand (L) zwischen der distalen Spitze der Probenstange (4) und der distalen Spitze der äußeren Kanüle (12) in dem Intervall von 0 - 3 mm liegt, wenn die Biopsie-Extraktions-Einheit (1) vollständig in die äußere Kanüle (12) eingeschoben ist.

## Revendications

1. Unité d'extraction de biopsie (1) pour son utilisation en relation avec un dispositif de biopsie (10) doté d'une canule externe (12), dans laquelle l'unité d'extraction de biopsie (1) est dotée d'une poignée unitaire (2) et d'une partie intermédiaire (3), dans laquelle l'unité d'extraction de biopsie (1) comprend en outre une tige d'échantillon allongée (4), dans laquelle la tige d'échantillon (4) est adaptée pour être positionnée essentiellement le long d'une paroi interne de la canule externe (12) lorsqu'elle est insérée dans ledit dispositif de biopsie (10), dans laquelle la tige (4) est agencée sur le plan distal de la partie intermédiaire (3), la tige (4) étant décalée et essentiellement parallèle à un axe longitudinal central (5) de l'unité d'extraction de la biospie (1),
**caractérisée en ce que** la tige d'échantillon (4) a une section transversale circulaire, elliptique ou quadratique ou dans laquelle la tige d'échantillon (4) a une section transversale triangulaire ayant un côté tourné vers l'extérieur par rapport à l'axe central (5) de l'unité (1) ou dans laquelle la tige d'échantillon (4) a une section transversale rectangulaire ayant un côté tourné vers l'extérieur par rapport à l'axe central (5) de l'unité (1), et
dans laquelle la tige d'échantillon (4) est configurée de façon à exercer une pression radiale sur un échantillon de tissu (13) contenu dans la canule externe (12) pour maintenir ledit échantillon (13) dans ladite canule (12) lorsque ladite tige d'échantillon (4) est insérée dans ledit dispositif de biopsie (10).

2. Unité d'extraction de biopsie (1) selon la revendication 1, dans laquelle la pointe distale de la tige d'échantillon (4) est acérée de sorte que le bord acéré soit plus éloigné de l'axe central (5) dans la direction radiale.

3. Unité d'extraction de biopsie (1) selon l'une quelconque des revendications précédentes, dans laquelle la tige d'échantillon (4) est parallèle à l'axe central (5) le long de toute sa longueur.

4. Unité d'extraction de biopsie (1) selon l'une quelconque des revendications précédentes, dans laquelle la partie distale de la tige d'échantillon (4) est courbée vers l'extérieur.

5. Unité d'extraction de biopsie (1) selon l'une quelconque des revendications précédentes, dans laquelle au moins la tige d'échantillon (4) est en métal.

6. Unité d'extraction de biopsie (1) selon l'une quelconque des revendications 1 à 4, dans laquelle au moins la tige d'échantillon (4) est en plastique.

7. Dispositif de biopsie comprenant une unité d'extraction de biopsie (1) selon l'une quelconque des revendications précédentes, et comprenant en outre une canule externe (12), dans lequel la longueur de la tige d'échantillon (4) est telle que lorsque l'unité d'extraction de biopsie (1) est complètement insérée dans la canule externe (12), la pointe distale de la tige d'échantillon (4) est située dans la canule externe (12).

8. Dispositif de biopsie selon la revendication 7, dans lequel la tige d'échantillon (4), lorsqu'elle est insérée dans la canule externe (12), couvre 3 à 15 % de la surface interne de la canule externe (12).

9. Dispositif de biopsie selon la revendication 7 ou 8, dans lequel la tige d'échantillon (4) a une largeur de section transversale maximale dans l'intervalle de 100 à 300 % de l'épaisseur de la paroi de canule de ladite canule externe (12), et dans lequel la tige d'échantillon (4) a une largeur de section transversale maximale dans l'intervalle de 10 à 20 % du diamètre externe de ladite canule externe (12).

10. Dispositif de biopsie selon l'une quelconque des revendications 7 à 9, dans lequel une distance (L) entre la pointe distale de la tige d'échantillon (4) et la pointe distale de la canule externe (12) est dans l'intervalle de 0 à 3 mm lorsque l'unité d'extraction de biopsie (1) est complètement insérée dans la canule externe (12).
